(19) Europäisches Patentamt

European Patent Office

Office européen des brevets

(11) Publication number: **0 273 771 B1**

# (12) EUROPEAN PATENT SPECIFICATION

(45) Date of publication of patent specification: **22.09.93**

(51) Int. Cl.5: **C12Q 1/02**, C12Q 1/18, //C12N5/00,G01N33/68

(21) Application number: **87311520.8**

(22) Date of filing: **30.12.87**

(54) **Mutagenic evaluation method.**

(30) Priority: **30.12.86 US 947669**

(43) Date of publication of application:
**06.07.88 Bulletin 88/27**

(45) Publication of the grant of the patent:
**22.09.93 Bulletin 93/38**

(84) Designated Contracting States:
**AT BE CH DE ES FR GB GR IT LI LU NL SE**

(56) References cited:
**WO-A-81/00726**
**WO-A-84/00384**

**CHEMICAL ABSTRACTS, vol. 106, no. 7, February 1987, Columbus, OH (US); F.S.SARIASLANI et al., p. 353, no. 47028n***

**CHEMICAL ABSTRACTS, vol. 96, no. 15, April 1982, Columbus, OH (US); K.ISHII et al., p. 173, no. 116872x***

**CHEMICAL ABSTRACTS, vol. 102, no. 11, 18 March 1985, Columbus, OH (US); P.LESCA et al., p. 142, no. 90876s***

**CHEMICAL ABSTRACTS, vol. 105, no. 11, 15**

**September 1986, Columbus, OH (US); G.MAZZA et al., p. 216, no. 92763p***

**CHEMICAL ABSTRACTS, vol. 98, no. 23, June 1983, Columbus, OH (US); D.M.MARON et al., p. 179, no. 192830p***

(73) Proprietor: **E.I. DU PONT DE NEMOURS AND COMPANY**
**1007 Market Street**
**Wilmington Delaware 19898(US)**

(72) Inventor: **Sariaslani, Fateme Sima**
**10 Colony Boulevard, No. 549**
**Wilmington, DE 19802(US)**

(74) Representative: **Hildyard, Edward Martin et al Frank B. Dehn & Co. European Patent Attorneys Imperial House 15-19 Kingsway London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a mutagenic evaluation method using cytochrome P-450 induced bacteria of the genus Streptomyces. We have found that Streptomyces enriched in their cytochrome P-450 concentration, e.g. using inducers such as soybean flour, genistein or genistin, are useful in carrying out a number of important chemical conversions including the conversion of procarcinogens into mutagens.

Cytochrome P-450 is a term for a group of unique heme proteins which form carbon monoxide complexes with a major absorption band at wavelengths around 450 nm. These proteins are isozymes which carry out oxidase functions in a wide variety of mixed function oxidase systems involved in biosynthesis and catabolism of specific cell or body components, and in the metabolism of foreign substances entering organisms. Oxygenating enzymes such as P-450 appear to be fundamental cellular constituents in most forms of aerobic organisms. The activation of molecular oxygen and incorporation of one of its atoms into organic compounds catalyzed by these enzymes are reactions of vital importance not only for biosynthesis and degradation of steroid hormones necessary for sustaining life, but also for metabolic activation or inactivation of foreign agents such as drugs, food preservatives and additives, insecticides, carcinogens and environmental pollutants.

Cytochrome P-450 enzymes can be induced in bacteria from the genus Streptomyces by e.g. a soybean derivative such as soybean flour, or the isoflavonoids genistin or genistein. (See e.g. Chemical Abstracts , Vol. 106, no. 7, February 1987 , page 353, abstract no. 47028n , by Sariaslani et al.) It has surprisingly been found that such bacteria enriched in cytochrome P-450 are useful in carrying out mutagenicity assays. The P-450 enzymes in such enriched bacteria bear a remarkable resemblance, in their oxidative reactions, to the cytochrome P-450 enzymes of mammalian liver and thus may serve as an economical and convenient source of cytochrome P-450 for use in a modified version of an Ames-type Salmonella assay for carcinogenicity and mutagenicity. Although cytochrome P-450 isozymes are found in a wide variety of organisms and tissues, they vary greatly in their composition and functions they can perform. Thus, it is highly surprising that the cytochrome P-450 enzymes of Streptomyces carry out a spectrum of oxidative reactions that are sufficiently similar to those carried out by the cytochrome P-450 enzymes of the mammalian liver that they may serve as a substitute in the Ames test.

The invention provides a method for evaluating the potential mutagenic activity of a substance which comprises:

(a) inducing the production of cytochrome P-450 in Streptomyces griseus by culturing said bacteria in a medium containing soybean flour or a soybean derivative;
(b) culturing a sample of Streptomyces griseus bacteria obtained from step (a) in a culture medium comprising the substance to be evaluated;
(c) incubating an amino acid requiring mutant strain of Salmonella typhimurium in the presence of a sample of bacterial culture from (b); and
(d) determining the number of resulting amino acid sufficient revertant colonies.

The process of the invention is carried out by culturing bacteria of the genus Streptomyces in a culture medium containing a cytochrome P-450 inducer. Suitable bacteria include, but are not limited to, Streptomyces griseus ATCC No. 13273, Streptomyces griseus ATCC No. 10137, Streptomyces griseus ATCC No. 13968, Streptomyces griseus ATCC No. 21897, Streptomyces griseus NRRL No. B-8090, Streptomyces punipalus NRRL No. 3529 and Streptomyces griseolus ATCC No. 11796, and combinations thereof. Preferably the bacteria utilized are selected from the group consisting of Streptomyces griseus ATCC No. 13273, Streptomyces griseus ATCC No. 10137, Streptomyces griseus ATCC No. 13968, Streptomyces griseus NRRL No. B-8090, Streptomyces punipalus NRRL No. 3529 and Streptomyces griseolus ATCC No. 11796. More preferably, the bacteria are selected from the group consisting of Streptomyces griseus ATCC No. 13273, Streptomyces griseus ATCC No. 10137, and Streptomyces griseus NRRL No. B-8090. Most preferably, the bacteria are Streptomyces griseus ATCC No. 13273. As used herein, the designation "ATCC" refers to the American Tissue Culture Collection depository located in Rockville, Maryland. The "ATCC No." is the accession number to cultures on deposit at the ATCC. As used herein, the designation "NRRL" refers to the U.S. Department of Agriculture, Northern Regional Research Laboratories, located in Peoria, Illinois, and the "NRRL No." is the accession number to cultures on deposit at the NRRL. In addition, Streptomyces bacteria suitable for use in the present induction process are also found in most soils and can be isolated therefrom using conventional methods which are apparent to those skilled in the art.

Contemplated inducers include soybean derivatives such as soybean flour, Bacto-soytone™, soypeptone™, genistein, genistin, and combinations thereof. Preferably, the inducer is soybean flour, Bacto-soytone™, soypeptone™ or genistein. More preferably, the inducer is soybean flour or genistein. Most preferably, from an activity standpoint, the inducer is genistein. As used herein, soybean flour includes both finely ground soybean powder and coarsely ground soybean meal. Preferably, the soybean flour is a finely ground powder. Soybean flour is commercially available from Natural Sales Co., P.O. Box 25, Pittsburgh, PA 15230 and Sigma Chemical Co., P.O. Box 14508, St. Louis, MO 63178. Bacto-soytone™ can be obtained from Difco Labs, Detroit MI, 28232 and Soypeptone™ from Sigma Chemical Co. Genistein is available from K&K Labs, Plainview, NY 11803. Soybean flour, genistein and genistin can each be obtained from soybeans using conventional methods which are apparent to those skilled in the art.

As will be clear to those skilled in the art, the amount of inducer required will vary depending upon the inducer utilized, the bacterial species and strain employed, the particular nutrient medium used, and the extent of P-450 production desired. In general terms, the higher the concentration of inducer, the greater the amount of cytochrome P-450 enzyme produced. Relatively speaking, high levels of cytochrome P-450 enzymes can be induced using about 2.5 to 25 g/L of soybean flour, Bacto-soytone™ or Soypeptone™, or about 50 to 150 mg/L of genistin or genistein.

The culturing can be carried out in any liquid culture medium suitable for the growth of bacteria of the genus Streptomyces. Such media are well known in the art. Generally, the medium should contain, glycerol, glucose, yeast extract or soybean flour, and have a pH of about 5 to 8, with the optimum pH falling near 7.0. Preferably the medium contains soybean flour or glycerol. Most preferably, the medium contains soybean flour. Suitable buffer salts include potassium or sodium phosphate or potassium or sodium chloride.

Examples of suitable media include the following: (1) glycerol (20 g/L), yeast extract (5 g/L), NaCl (5 g/L), $K_2HPO4$ (5 g/L) and distilled water, with the pH adjusted to 7.0 using 5N HCl; (2) sporulation broth [ATCC No. 5] consisting of yeast extract (1.0 g/L), beef extract (1.0 g/L), tryptose (2.0 g/L), $FeSO_4$ (trace), glucose (10 g/L) and distilled water, with the pH adjusted to 7.2 using 1N NaOH; and (3) nutrient broth consisting of bacto beef extract (3 g/L), bacto peptone (5 g/L) and distilled water. Other suitable media are described in Koenig et al., Helv. Chim. Acta, 60: 2071-8 (1977).

Generally, the cultures should be maintained at temperatures between about 20-37°, preferably between about 25-37°, with the optimum growth temperature at about 28-30°.

A suitable culturing procedure requires growing the bacteria in a suitable culture medium containing one or more of the inducers for up to about 5 days at a temperature between about 25 and 37°, and transferring the bacteria into a fresh inducer-containing medium maintained within the same temperature range. The bacteria are then maintained in the fresh medium for up to about 5 days. The period of growth prior to a transfer into fresh inducer-containing medium is termed "stage 1", and the organisms from this period of growth are called "first stage" organisms. The growth period after the transfer of organisms from the first stage into new inducer-containing medium is termed "stage 2" and the organisms from this stage called "second stage" organisms. This culturing procedure is termed a two-stage culturing protocol. The most preferred culturing procedure is carried out by growing the bacteria in stage 1 at 28-30°C for 3 days, transferring the bacteria to fresh medium and growing in stage 2 for one additional day at the same temperatures. Preferably the cultures in stages 1 and 2 are incubated with shaking.

The bacteria can then be harvested from the bacterial culture by centrifugation. Centrifugation rates of 8,000 - 15,000 x g for 15-30 minutes can be employed to yield a cellular pellet portion and a substantially cell-free supernatant. Preferably, a rate of 9,000 x g for 20 minutes is used.

Cytochrome P-450 induced Streptomyces bacteria may be employed to determine the mutagenicity of a given substance, and the present invention also encompasses this. As used herein, mutagenicity is defined as including both mutagenicity and carcinogenicity. Specifically, the present invention contemplates a method for evaluating the potential mutagenic activity of a substance which comprises: (a) culturing cytochrome P-450 induced bacteria from the genus Streptomyces in a culture medium comprising the substance to be evaluated; (b) incubating an amino acid requiring mutant strain of Salmonella typhimurium in the presence of a sample of the bacteria culture from (a); and (c) determining the number of resulting amino acid sufficient revertant colonies.

Cytochrome P-450 induced Streptomyces bacteria for the mutagenicity assay may be obtained using the induction procedures described in detail above. The culture conditions such as culture medium, temperature, pH, etc. utilized in the induction process described herein are also suitable for carrying out step (a) of the mutagenicity assay.

Preferably, the induced bacteria utilized in the mutagenicity assay are produced using the two-stage culturing protocol, and step (a) of the mutagenicity assay is carried by adding the substance to be evaluated directly to the second stage culture medium containing the induced bacteria, as described in Example 38 below. Most preferably, the substance to be evaluated is added to the second stage culture medium at about 24 hr, as described in Examples 39-46 below. Other suitable variations within the ambit of the invention as described in step (a) will be apparent to one skilled in the art.

The genus and strain of the induced Streptomyces bacteria employed in step (a) are not critical. Suitable induced Streptomyces bacteria include, but are not limited to, induced Streptomyces griseus ATCC No. 13273, induced Streptomyces griseus ATCC No. 10137, induced Streptomyces griseus ATCC No. 13968, induced Streptomyces griseus ATCC No. 21897, induced Streptomyces griseus NRRL No. B-8090, induced Streptomyces punipalus NRRL No 3529 and induced Streptomyces griseolus ATCC No. 11796. Preferably, the induced Streptomyces bacteria employed are selected from the group consisting of induced Streptomyces griseus ATCC No. 13273, induced Streptomyces griseus ATCC No. 10137, induced Streptomyces griseus ATCC No. 13968, induced Streptomyces griseus NRRL No. B-8090, induced Streptomyces punipalus NRRL No. 3529 and induced Streptomyces griseolus ATCC No. 11796. More preferably, the induced Streptomyces bacteria are selected from the group consisting of induced Streptomyces griseus ATCC No. 13273, induced Streptomyces griseus ATCC No. 10137 and induced Streptomyces griseus NRRL No. B-8090. Most preferably, the induced Streptomyces bacteria are induced Streptomyces griseus ATCC No. 13273.

The bacterial culture sample utilized in step (b) may be obtained by taking a sample directly from the bacterial culture in step (a). Alternatively, the bacterial culture in (a) may be centrifuged to obtain a cellular pellet portion and a substantially cell-free supernatant portion, and either portion used as the bacterial culture sample in step (b). Suitable centrifugation rates include 8,000 to 15,000 x g for 15 to 30 minutes. Preferably, the centrifugation rate is 9,000 x g for 20 minutes.

Incubation conditions suitable for step (b) of the mutagenicity assay will be apparent to one skilled in the art, and are described in Maron et al., Mutation Research, 113: 178-179 (1983), and in Examples 38-46 below. In this regard reference may also be made to Ames et al., Proc. Natl. Acad. Sci., 70:2281 (1973); Ames et al., Mutation Research, 31:347 (1975); McCann et al., Proc. Natl. Acad. Sci. USA, 72:5135 (1975); and McCann et al., Proc. Natl. Acad. Sci. USA, 73:950 (1976).

Any amino acid requiring mutant strain of Salmonella typhimurium suitable for use in the well-known Ames mutagenicity assays may be employed in the present mutagenicity assay. Preferably the amino acid requiring mutant strain of Salmonella typhimurium is a histidine requiring mutant strain. Such Salmonella are discussed, for example, in Maron et al., Mutation Research, 113: 178-179 (1983), which describes a revised Ames assay. Suitable histidine requiring mutant strains include, but are not limited to strains TA 97, TA 97A, TA 98, TA 100, TA 100FR, TA 102, TA 104, TA 1535, TA 1537, TA 1538 and M 677. Preferably, the mutant strain of Salmonella typhimurium is selected from the group consisting of strains TA 98, TA 1535, TA 1537 and TA 1538. Most preferred, the mutant strain of Salmonella typhimurium is selected from the group consisting of strains TA 1535, TA 1537 and TA 1538. On deposit with the ATCC and bearing the following ATCC accession are the following strains of histidine requiring Salmonella typhimurium: Salmonella typhimurium, strain TA 1535, ATCC No. 29629; Salmonella typhimurium, strain TA 1537, ATCC No. 29630; and Salmonella typhimurium, strain TA 1538, ATCC No. 29631. On deposit with the NRRL and bearing the following NRRL accession numbers are the following strains of histidine requiring Salmonella typhimurium: Salmonella typhimurium, strain TA 98, NRRL No. B-4279; Salmonella typhimurium, strain TA 100, NRRL No. B-4280; Salmonella typhimurium, strain TA 1535, NRRL No. B-4211; Salmonella typhimurium, strain TA 1537, NRRL No. B-4213; and Salmonella typhimurium, strain TA 1538, B-4214. A number of these and other mutant strains of Salmonella typhimurium are also available upon request from Bruce Ames, Biochemistry Department, University of California, Berkeley, CA 94720. In addition, these and other mutant strains suitable for use in the present mutagenicity assay may be constructed using recombinant DNA techniques well- known to those skilled in the art.

The number of resulting amino acid sufficient revertant colonies in step (c) can be determined using techniques well-known to those skilled in the art.

In addition, cytochrome P-450 induced Streptomyces bacteria may be utilized in carrying out many commercially important oxidation reactions, as will be recognized by those skilled in the art.

The compounds which may be oxidized by the cytochrome P-450 induced Streptomyces bacteria (and the oxidized compound resulting therefrom) include the following: 7-ethoxycoumarin (7-hydroxycoumarin); precocene II (precocene-diol); anisole (phenol, 2-OH anisole); benzene (phenol); biphenyl (4-OH biphenyl); chlorobenzene (2-OH chlorobenzene); coumarin (7-OH coumarin); naphthalene (1-OH naphthalene); trans-stilbene (4-OH stilbene, 4,4'-di-OH stilbene); toluene (2-OH toluene); glaucine (predicentrine, norglaucine); 10,11-dimethoxyaporphine (apocodeine, isoapocodeine); papaverine (6-desmethylpapaverine, 7-desmethyl-papaverine, 4'-desmethylpapaverine); d-tetrandrine (N'-nortetrandrine); thalicarpine (hernandalinol); brucean-tin (side chain alcohols, epoxide); vindoline (dihydrovindoline ether, dihydrovindoline ether dimer, dihydrovindoline ether enamine); dihydrovindoline (11-desmethyldihydrovindoline); leurosine (12'-hydrox-yleurosine); and codeine (14-hydroxycodeine).

The invention is further described in the Examples set forth below. All experiments were conducted in accordance with the following General Methods, except where otherwise indicated. In the General Methods and Examples, all parts and percentages are by weight, and all degrees are Celsius unless otherwise noted.

Aspects of Examples 26 and 32 are illustrated in the accompanying drawings, wherein:

Figure 1 illustrates the FPLC profiies of ammonium sulfate pellets of S. griseus extracts grown on soybean medium, as described in Example 26. Figure 1(A) represents the FPLC analysis of a 35-45% pellet fraction and Figure 1(B) represents the FPLC analysis of a 45-55% pellet fraction.

Figure 2 shows the SDS-page gel electrophoresis of S. griseus extracts, as described in Example 32.

## General Methods

### Chemicals

The soybean flour routinely used in these studies was purchased from Natural Sales Co., P.O. Box 25, Pittsburgh, PA 15230. Soybean flour may also be obtained from Sigma Chemical Co., P.O. Box 14508, St. Louis, MO 63178.

Genistein was purchased from K&K Labs., Plainview, NY 11803.

Soy oil was purchased from Sigma Chemical Co., P.O. Box 14508, St. Louis, MO 63178.

Coumestrol was purchased from Eastman Kodak Co., Rochester, NY 14650.

Daidzein was purchased from Spectrum Chemical MFG Corp., 14422 S. San Pedro St., Gardena, CA 90248.

### Bacterial Strains

Streptomyces griseus (ATCC 13273, ATCC 10137, ATCC 13968, ATCC 21897, NRRL B-8090, ATCC 23336 and NRRL 3242).

Streptomyces punipalus (NRRL 3529).

Streptomyces griseolus (ATCC 11796).

Streptomyces lincolnensis (ATCC 25466).

### Culture Maintenance

Bacteria were maintained on sporulation agar stock slants or plates and stored in sealed screw-capped tubes at 4°.

### Media and Growth Conditions

The bacteria were grown on three different complex media. "Medium A" contains soybean flour (5 g), glycerol (20 g), yeast extract (5 g), NaCl (5 g), $K_2HPO4$ (5 g), and distilled water (1.0 L); with the pH adjusted to 7.0 with 5N HCl. "Medium B" is sporulation broth (ATCC No. 5) and consists of yeast extract (1.0 g), beef extract (1.0 g), tryptose (2.0 g), $FeSO_4$ (trace), glucose (10 g) and distilled water (1.0 L); with the pH adjusted to 7.2 using 1N NaOH. "Medium C" is nutrient broth. Nutrient broth consists of bacto beef extract (3 g) and bacto peptone (5 g) in 1.0 L of distilled water. All media were sterilized by autoclaving.

The bacteria were grown according to a two stage fermentation protocol as described by R. E. Betts et al., J. Med. Chem. 17:599-602, (1974). Bacteria were transferred from stock slants or plates to either 25 mL or 200 mL of the relevant media and incubated with shaking (2500 rpm) at 28-30° for three days (stage 1). A portion of the stage 1 cultures sufficient to constitute 10-20% of the volume of the new culture was used to start stage 2 cultures. Stage 2 cultures were usually incubated with shaking for 24-72 h, preferably 24 h.

### Preparation of Cell Suspensions and Cell Extracts

Bacteria grown on liquid medium were harvested by centrifugation (9,000 x g, 20 min) and washed once with one volume of buffer (pH 7.4) containing 100 mM sodium phosphate, 1 mM dithiothreitol (DTT) and 0.1 mM ethylene diaminetetraacetic acid (EDTA). This buffer is called DEP buffer. Cell suspensions in DEP buffer were prepared by suspending 5-8 g (wet weight) of centrifuged bacteria in 15-24 mL of buffer. The bacteria were disrupted by two passages through a French Pressure cell at $8.27 \times 10^6$ Pa (1200 psi) pressure. The resulting viscous liquids (19-29 mL) were digested with deoxyribonuclease (5 mg/ 20 mL of extract) for 7 min at about 25° and centrifuged at 105,000 x g for 1 h. When there was a white film at the top of 105,000 x g supernatant fluid, it was discarded and the remaining clear yellow supernatant fluids containing the cytochrome P-450 enzymes were used. In some cases (see, e.g., Example 30) instead of centrifuging at 105,000 x g for 1 hr, the digested liquids were centrifuged at 50,000 x g for 1 h and the resulting supernatant fluids were used.

## Total Protein Content Assay

The total protein content of the extracts was determined by the method of Lowry et al., J. Biol. Chem., 193:165-275, (1951).

## Spectrophotometric Cytochrome P-450 Assay
## (CO-Difference Spectrophotometry)

Cytochrome P-450 was assayed by the method of Omura and Sato, J. Biol. Chem., 239:2370-2378, (1964), using a Perkin Elmer lambda 5 recording spectrophotometer. The extract (1 mL) was placed in both sample and reference cuvettes. The sample cuvette was reduced by the addition of a few crystals of sodium dithionite and the spectrum was recorded. Then the reference cuvette was reduced in the same fashion, and carbon monoxide was bubbled through the sample cuvette and the spectrum recorded. The O.D. at 448 nm was measured and the concentration of P-450 was calculated using a molar extinction coefficient of 91 mM$^{-1}$ x cm$^{-1}$ for the chromophore.

## Quantitative Analysis of Genistein

These analyses were performed using a Du Pont 850 High Pressure Liquid Chromatograph (HPLC). Two solvent systems were used. Solvent A consisted of water and acetic acid in a ratio of 100:0.1 (v/v). Solvent B consisted of methanol and acetic acid in a ratio of 100:0.1 (v/v). A Du Pont C8 column was employed for the analyses and the injected samples were eluted with a mixture of 30% A and 70% B maintained isocratically at a flow rate of 1 mL x min$^{-1}$. The concentration of genistein in the samples was estimated using a calibration curve obtained from analysis of known quantities of genistein. Samples of cultures (2 ml) were mixed with ETOAC (1 mL), shaken vigorously and centrifuged. The organic layer (0.5 ml) was then dried under nitrogen, dissolved in 300 $\mu$L of MeOH, and analyzed by High Pressure Liquid Chromatography (HPLC). Using this system, genistein was eluted at 6.2 mL.

## Analysis of Proteins by Gel Electrophoresis

Proteins in the 105,000 x g supernatant fluids and desalted ammonium sulfate fractions thereof were analyzed by SDS-page electrophoresis (SDS = sodium dodecyl sulfate). For this purpose, 20$\mu$L of sample containing approximately 5 mg per mL protein was added to 10 $\mu$L of SDS solubilizing buffer and heated at 95° for 3 min. SDS solubilizing buffer consists of 150 mM Na tricine (pH 7.8), obtained from Sigma Chemical Co., 150 mM dithiothreitol, 21% (w/v) glycerol 0.003% (w/v) bromphenol blue, and 6% lithium dodecyl sulfate. The entire 30 $\mu$L sample was then loaded onto a 15 x 32 cm 8% (or 10-17% gradient) polyacrylamide slab gel and electrophoresed overnight at 5-10 watts. After electrophoresis the gel was removed from the casting apparatus and the protein bands were stained with Coummassie blue.

## Preparation of Phosphate Buffer Saline Solution (PBS w/Ca + + and Mg + + )

The following ingredients were used to prepare the buffer: calcium chloride (CaCl$_2$ 2H$_2$O; 0.7 mM); potassium chloride (KCl; 2.7 mM); potassium phospate monobasic (KH$_2$PO$_4$; 1.5 mM); magnesium chloride-(MgCl$_2$ 6H$_2$O; 0.5 mM); sodium phosphate dibasic (Na$_2$HPO$_4$ 7H$_2$O); 8.1 mM); and sodium chloride (NaCl; 13.7 mM). The buffer was prepared by adding the following amounts of these ingredients, in the following order, to a suitable vessel containing 10 L of distilled, deionized water: CaCl$_2$ 2H$_2$O - 1.0 g; KCl - 2.0 g; KH$_2$PO$_4$ - 2.0 g; NaCl - 8.0 g; MgCl$_2$ 6H$_2$O - 1.0 g; and Na$_2$HPO$_4$ 7H$_2$O - 21.6 g. The mixture was stirred until all components were dissolved, and the solution then filter sterilized with a Millipore filter apparatus directly into 500 mL sterile screw-cap bottles. Next, 0.1 mL of the filtered solution was aseptically streaked on a complete base agar plate and the plate incubated for 24 hr at 37° to sterilize.

### Preparation of Minimal Base Agar Plate

To prepare the minimal base agar plates, 60 g of Difco Bacto agar (granular), obtained from Difco Labs, Detroit, MI 48232, and 92.8 g of Davis Minimal Broth (dry form), obtained from Gibco Labs, Grand Island, NY 14072, were suspended in 4000 mL of distilled, deionized water. The suspension was then stirred with a Teflon™ coated magnetic stirrer until the Minimal Broth was dissolved, and a sponge plug was placed in the top of flask and the flask covered with aluminum foil. The flask was then autoclaved for 20 minutes (15 psi, 121°). The flask was removed from the autoclave and, after 30 min, the contents stirred and dispensed onto a petri dish (25 mL per 100 x 15 mM petri plate (Falcon™ 1029) using Petrimat™ plate filling equipment. The filled petri plates were allowed to cool and stored at 4°.

### Preparation of Complete Base Agar Plate

To prepare the complete base agar plates, 94.0 g of BBL Standard Methods agar (2.4%) (which contains, per liter, 5 g of pancreatic digest of casein, 2.5 g of yeast extract, 1 g of dextrose and 15 g of agar) was suspended in 4000 mL of distilled, deionized water. The suspension was stirred with a Teflon™ coated magnetic stirrer until the Standard Methods agar was dissolved, and a sponge plug was placed in the top of flask and the flask covered with aluminum foil. The flask was then autoclaved for 60 min (15 psi, 121°). The flask was removed from the autoclave and, after 30 min, the contents stirred and dispensed onto a petri dish (25 mL per 100 x 15 mM petri plate (Falcon™ 1029) using Petrimat™ plate filling equipment. The filled petri plates were incubated at 37° for 24 hr to check sterility and then stored at 4°.

### Preparation of 0.5 mM L-Histidine/0.5mM Biotin Solution

Approximately 450 mL of distilled, deionized water was added to a 500 mL volumetric flask containing 61.0 mg of D-biotin and 52.5 mg L-histidine, both obtained from Sigma Chemical Co., P.O. Box 14508, St. Louis, MO. The flask was stirred until all components were dissolved and the flask brought to volume. The solution was then filter sterilized with a Nalgene 0.2 micron filter unit, produced by Nalgene Labware, Nalge Co., Rochester, NY 14602. Next, 0.1 mL of the filtered solution was aseptically streaked on a complete base agar plate, the plates were incubated for 24 hr at 37° to check sterility, and 100 mL of the solution dispensed into each of five 100 mL bottles for storage at 4°.

### Preparation of Top Agars

Standard top agar was prepared by first suspending 24.0 g of Difco Bacto agar and 24.0 g of sodium chloride (NaCl) in 4000 mL of distilled, deionized water. The solution was mixed and sterilized by autoclaving for 20 min at 121° (15 psi) and 75 mL dispensed into 100 mL capacity screw-cap bottles for storage at room temperature.

### Procedure for Growing Cultures of Salmonella Strains

Suitable procedures for growing Salmonella cultures are well known in the art, and the particular procedure employed is not critical. The procedure employed in the Examples below is set forth in Maron et al., Mutation Research, 113: 178-179 (1983).

### EXAMPLES

### Examples 1-10

Following the growth conditions described in the General Methods, organisms were transferred from sporulation agar stock slants or plates to 25 mL of Medium A and incubated with shaking at 2500 rpm at 28-30° for three days--i.e., stage 1. Each strain was grown as a separate culture. Stage 2 cultures of each strain were prepared by inoculating 200 mL of medium A with 20 mL of stage 1 culture. Each stage 2 culture was incubated for 24 hours under the conditions used for stage 1 cultures. Each culture was processed separately and 105,000 x g cell-extracts prepared separately using the Preparation of Cell Suspension and Cell Extracts procedures described in the General Methods. The yellow supernatant fluids were then assayed for cytochrome P-450 material using CO-Difference Spectrophotometry as discussed in the General Methods. The results are summarized below (Table 1).

Table 1

| Example | Medium | Organism | pmol P-450 per mg protein[*] |
|---------|--------|----------|------------------------------|
| 1 | A | S. griseus, ATCC 13273 | 122.5 |
| 2 | A | S. griseus, ATCC 10137 | 28.2 |
| 3 | A | S. griseus, ATCC 13968 | 10.2 |
| 4 | A | S. griseus, ATCC 21897 | 2.3 |
| 5 | A | S. griseus, NRRL B-8090 | 53.6 |
| 6 | A | S. punipalus, NRRL 3529 | 6.2 |
| 7 | A | S. griseolus, ATCC 11796 | 5.3 |
| 8 | A | S. griseus, ATTC 23336 | <2.0 |
| 9 | A | S. griseus, NRRL 3242 | <2.0 |
| 10 | A | S. lincolnensis, ATCC 25466 | <2.0 |

[*] <2.0 means below measurable amounts

The above data illustrates the production of measurable amounts of cytochrome P-450 for a number of Streptomyces species and strains grown as described in medium A. The greatest amount of P-450 enzymes was produced by S. griseus, ATCC 13273. Although S. griseus, ATTC 23336, S. griseus, NRRL 3242, and S. lincolnensis, ATCC 25466 failed to produce measurable amounts of P-450 enzymes in this experiment, it is believed that at higher inducer concentrations, measurable amounts would be present.

Examples 11 - 16

The procedures of Examples 1-10 were repeated except that the organisms were grown on Medium B (sporulation broth) and on medium C (nutrient broth). Only the three strains of Streptomyces producing the greatest amounts of cytochrome P-450 enzymes were used, namely, S. griseus ATCC 13273, ATCC 10137, and NRRL B-8090.

8

Table 2

| Example | Medium | Organism | pmol P-450 per mg protein* |
|---------|--------|----------|----------------------------|
| 11 | B | S. griseus, ATCC 13273 | <2.0 |
| 12 | C | S. griseus, ATCC 13273 | <2.0 |
| 13 | B | S. griseus, ATCC 10137 | <2.0 |
| 14 | C | S. griseus, ATCC 10137 | <2.0 |
| 15 | B | S. griseus, NRRL B-8090 | <2.0 |
| 16 | C | S. griseus, NRRL B-8090 | <2.0 |

*<2.0 means below measurable amounts

As the data shows, the tested bacteria failed to produce measurable amounts of cytochrome P-450 enzymes on either media.

Examples 17 - 22

The procedures of Examples 1-10 were repeated except that cultures of S. griseus, ATCC 13273, were grown in the following media: Medium A having 5 g/L of soy oil substituted for the 5 g/L of soybean flour; Medium B with 5 g/L of soybean flour added; Medium B with 0.1 g/L of genistein added; Medium B with 0.1 g/L of coumestrol added; Medium B with 0.1 g/L of daidzein added; or Medium C with 5 g/L of soybean flour added. The amounts of cytochrome P-450 enzymes produced by each of comparable cultures is shown in Table 3. Soy oil, soybean flour, genistein, coumestrol and diadzein are all soybean derivatives. Soy oil, while containing many of the substituents of soybeans does not contain genistein.

Table 3

| Example | Medium | Substitution or Addition | pmol P-450 per mg protein* |
|---------|--------|--------------------------|----------------------------|
| 17 | A | with 5.0 g/L soy oil substituted for 5.0 g/L soybean flour | <2.0 |
| 18 | B | with 5.0 g/L soybean flour added | 28.0 |
| 19 | B | with 0.1 g/L genistein added | 124.8 |
| 20 | B | with 0.1 g/L coumestrol added | <2.0 |
| 21 | B | with 0.1 g/L daidzein added | <2.0 |
| 22 | C | with 5.0 g/L soybean flour added | 8.6 |

*<2.0 means below measurable amounts

Cytochrome P-450 enzymes were produced whenever soybean flour or genistein were present regardless of the media. Media without soybean flour or genistein but containing soy oil, coumestrol, or daidzein, all of which are soybean substituents, failed to induce cytochrome P-450 enzymes.

Examples 23 - 25

The procedures of Examples 1-10 were repeated except that cultures of S. griseus, ATCC 10137 were grown in the following media with the following added constituents: Medium B containing 5 g/L of soybean flour, or Medium B containing 0.1 g/L of genistein, or Medium C containing 5 g/L of soybean flour. The amounts of cytochrome P-450 enzymes produced by each culture are shown in Table 4.

Table 4

| Example | Medium | Addition | pmol P-450 per mg protein |
|---------|--------|----------|---------------------------|
| 23 | B | soybean flour (5.0 g/L) | 6.1 |
| 24 | B | genistein (0.1 g/L) | 20.2 |
| 25 | C | soybean flour (5.0 g/L) | 4.9 |

Cytochrome P-450 enzymes were produced whenever soybean flour or genistein were present regardless of the medium.

10

Example 26

The procedures of Examples 1-10 were repeated except that a culture of S. griseus, NRRL B-8090 was grown in Medium B containing 0.1 g/L of genistein. The amount of cytochrome P-450 enzymes produced is shown in Table 5.

**Table 5**

| Example | Medium | Addition | pmol P-450 per mg protein |
|---------|--------|----------|---------------------------|
| 26 | B | genistein (0.1 g/L) | 60.0 |

Significant cytochrome P-450 enzymes were produced.

From the data in Examples 1 through 26, it is clear that soybean flour and soybean flour substituents genistein and genistin can induce the production of cytochrome P-450 enzymes in bacteria from the genus Streptomyces.

Example 27

A three-day old stage 1 culture (200 mL) of S. griseus ATCC 13273 grown using the procedures in Examples 1-10 in Medium B was centrifuged and resuspended in 200 mL of fresh Medium B. The resulting suspension was added to a 1500 mL capacity fermenter vessel containing 1200 mL of the medium. Genistein (120 mg) was dissolved in about 3-4 mL of solvent consisting of dimethylformamide and methanol in a ratio of 3:2 (v/v), dimethylformamide:methanol, and the solution was added to the medium. Samples of 100-200 mL were removed periodically and their dry weight and cytochrome P-450 contents determined. The dry weight of each sample was measured by filtering 5 mL of the sample through a preweighed Nuclepore PC membrane (47 mm, 4μm) filter and drying it overnight at 60°. The contents of cytochrome P-450 of each sample was determined essentially as in Examples 1-10. The results are shown in Table 6.

**Table 6**

| Time (h) | Dry wt. (mg/mL) | pmol P-450 per mg protein* |
|----------|-----------------|----------------------------|
| 0 | 0.326 | 3 |
| 7.5 | 0.314 | 12.5 |
| 23.5 | 0.408 | 30.3 |
| 48 | 1.54 | 56 |
| 72 | 1.18 | 19.3 |
| 96 | 1.3 | <2.0 |

*<2.0 means below measurable amounts

The results indicate that cytochrome P-450 is induced during the growth lag phase, reaches a maximum level between 48 and 72 h, after which it diminishes and finally disappears at 96 h. HPLC analysis of the supernatant fluids of batch cultures (shake flasks) containing genistein, conducted as described in the General Methods, Quantitative Analysis of Genistein section, indicate that during the

11

induction and disappearance of cytochrome P-450, utilization of genistein does not occur.

Example 28

A stage 2 culture of S. griseus, ATCC 13273 grown for 24 h in Medium A was harvested yielding 57.6 g wet weight of cells. An extract of these cells was obtained as described in Examples 1-10. After centrifugation at 105,000 x g, a supernatant fluid of 102 mL was obtained. The supernatant fluid was fractionated by ammonium sulfate precipitation as follows. To obtain 35% of the saturation concentration of ammonium sulfate, 20.8 g of ammonium sulfate crystals were added to each 100 mL of the crude extract during a period of about 15 min with constant slow stirring. When the entire amount of salt was added, the stirring was continued for an additional 10 min to allow complete equilibration between dissolved and aggregated proteins. The cloudy liquid was centrifuged for 30 min at 18,000 x g to separate the aggregated proteins. The pellet obtained in this fashion was labeled "0-35% pellet" and the supernatant fluid was labeled "0-35% supernatant". To each 100 mL of the "0-35% supernatant", 6.16 g of ammonium sulfate crystals were added, essentially as above, to obtain 45% of the saturation concentration. The cloudy solution was centrifuged, as above, to obtain the "35-45% pellet" and the "35-45% supernatant". A "45-55% pellet" and "45-55% supernatant" was obtained by adding, essentially as above, 6.38 g of ammonium sulfate crystals to each 100 mL of the "35-45% supernatant" followed by centrifugation, as above. The P-450 content of each of these fractions was determined as previously described in Examples 1-10. The total protein was determined in accordance with the General Methods, Total Protein Content Assay section. The pmol per mg protein was then calculated from the total protein and P-450 determinations. The results are summarized in Table 7.

## Table 7

| Sample | Protein (mg/mL) | P-450 (pmol/mL) | pmol P-450 per mg protein |
|---|---|---|---|
| Crude | 12.5 | 749.4 | 59.9 |
| 0-35% supernatant | 7.4 | 560.0 | 75.6 |
| 0-35% pellet | 5.5 | 98.9 | 17.9 |
| 35-45% supernatant | 5.3 | 149.0 | 28.1 |
| 35-45% pellet | 38.4 | 5934.0 | 154.5 |
| 45-55% supernatant | 3.3 | 0.0 | 0.0 |
| 45-55% pellet | 45.4 | 1560.0 | 34.4 |

From the above data it is clear that the majority (almost 80%) of the cytochrome P-450 containing material is precipitated in the fraction resulting when the ammonium sulfate concentration was increased from 35% to 45% of the saturation level.

Example 29

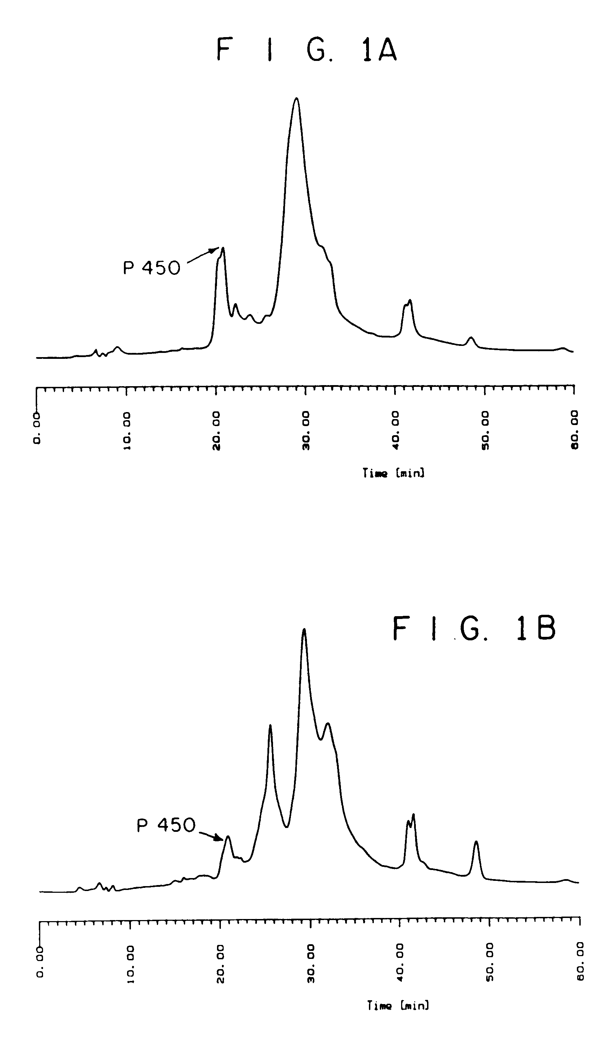

The 35-45% and 45-55% pellets of Example 28 were analyzed by Fast Protein Liquid Chromatography (FPLC) as follows. Sephadex® G-25 (0.5 g/ 50 mL), obtained from Sigma Chemical Co., P.O. Box 14508, St. Louis, MO 63178, was stirred in DEP buffer. Some of the resulting gel was added to a 1.0 mL syringe containing a porous plug. The syringe was placed in a centrifuge tube and centrifuged in a bench-top centrifuge at 3000 rpm for 3 min. The addition of gel followed by centrifugation was repeated until the column was almost filled with the gel. The column was washed with 0.5 mL of buffer. A sample (150 μL) of the ammonium sulfate pellet dissolved in DEP buffer was added to the top of this column followed by centrifugation as before. The desalted protein extracts that accumulated in the centrifuge tube were then analyzed by FPLC using an LKB instrument connected to a Hewlett Packard (HP) diode array spectrophotometer, an HP 85 computer, and an HP plotter. Analyses were done using an LKB glaspac TSK DEAE-3SW (8 x 75 mm) anion exchange column. The solvent systems were as follows: Solvent A consisted of 20 mM tris acetate and 20% glycerol (pH 7.4); Solvent B consisted of solvent A with 0.8 M sodium acetate added (pH 7.4). The gradient employed for the separation was 0-40 min from 0-100% B. Fractions were eluted at a flow rate of 0.75 mL per min and analyzed for cytochrome P-450 content as described above. The FPLC profiles at 420 nm can be seen in Figure 1. Specifically, Figure 1(A) shows the FPLC analysis of the 35-45% pellet fraction, and Figure 1(B) shows the FPLC analysis of the 45-55% pellet fraction. The analyses indicate that in both ammonium sulfate fractions, the fractions eluting at 15 mL contained the cytochrome P-450 material.

Example 30

Oxidation of precocene II and 7-ethoxycoumarin with cell-free extracts

S. griseus, ATCC 13273 was grown in 200 mL of Medium A as described in Examples 1-10 except that at the beginning of stage 2, 7-ethoxycoumarin (50 mg/ 200 mL) was added to one culture and precocene II (100 mg/200 mL) was added to another. Bacteria from these cultures were harvested after 24 h of incubation in stage 2 and subjected to the French Pressure cell as described in Examples 1-10. The broken cells were centrifuged at 18,000 x g for 10-15 min. The supernatant fluid of each was then centrifuged at 50,000 x g for 1 hr. The resulting supernatant fluid constituted the test extract. Preparative scale incubations were performed using the test extract (15-20 mL), reduced nicotinamide adenine dinucleotide phosphate (NADPH) (77-101 mg) as cofactor and either precocene II (0.51 mg dissolved in 0.5 mL of an equal mixture of ethanol and water) or 7-ethoxycoumarin (0.66 mg was dissolved in 0.5 mL of an equal mixture of ethanol and water). The resulting mixtures were incubated on the shaker at 28° for 1.5 h and then extracted with 1/2 volume of ethylacetate. The ethylacetate layer was dried under nitrogen and then taken up in a small volume of methanol for Thin Layer Chromatography (TLC) analysis. Products formed were isolated by preparative TLC using the solvent system of chloroform:methylene chloride:methanol:ethylacetate (10:10:1:1 v/v) for precocene metabolite, precocene-diol, and benzene:ethanol (45:5) for ethoxycoumarin metabolite, 7-hydroxycoumarin (umbelliferone). The bands corresponding to the precocene-diol and 7-hydroxycoumarin were then scraped off and the metabolites recovered from the silica gel by resuspending the scraped material in methanol and filtering the suspensions to remove the gel. The metabolites were recovered from the methanol by drying over anhydrous MgSO4, under reduced pressure.

The metabolites obtained in this fashion were derivatized by the addition of a couple of drops of tetramethylsilane (TMS) and were then analyzed by Gas Chromatography/ Mass Spectrometry (GC/MS). The TMS-derivatives of the metabolite isolated from the cell extract incubation with precocene II gave a molecular ion peak at m/e 398, a base peak at m/e 239 and another peak at m/e 73. The TMS-derivative of the isolated metabolite from incubation with 7-ethoxycoumarin gave a molecular ion peak at m/e 234, base peak at m/e 219, and major fragments at m/e 191, 163 and 73. These results are consistent with the GC/MS data for the TMS-derivatives of 7-hydroxycoumarin and trans-precocene-diol.

Example 31

In this example, a fluorometer was utilized to detect 7-ethoxycoumarin O-dealkylase activity. First, cells were grown on Medium A in the presence of ethoxycoumarin and an extract was prepared as described in Example 30. Enzyme assays were performed by first adding test extract (1-2 mg of protein) to a 2 mL reaction mixture containing 0.05 mM ethoxycoumarin and DEP buffer, and then initiating the reaction by the addition of reduced nicotinamide adenine dinucleotide (NADH) (0.5 mM) as a cofactor. The formation of 7-hydroxycoumarin was determined by irradiating the cuvette at 365 mM and recording the increase in light emission at 450 nm using a fluorometer. Using this approach the specific activity for 7-hydroxycoumarin formation from 7-ethoxycoumarin was measured at about 0.2 nmol x min$^{-1}$ x mg$^{-1}$ protein.

Examples 32 - 34

Cultures of S. griseus, ATCC 13273 were grown as described in Examples 1-10, except as follows. One culture was grown in Medium A, one culture in Medium B, and one culture in Medium B containing soybean flour at a concentration of 5 g/L. In addition, precocene II (100 mg/ 200 mL) was dissolved in 1 mL of methanol and added to the 24 h stage 2 cultures. Samples (3 mL) were withdrawn after 48 h. Each sample was extracted with ethylacetate (2 mL), thoroughly mixed, and centrifuged for 3 min in a benchtop clinical centrifuge. The ethylacetate layer (1 mL) was withdrawn, dried under a stream of nitrogen and dissolved in 0.3 mL of methanol prior to HPLC analysis.

Quantitative analyses of precocene-diol formation were performed using a Du Pont 850 HPLC connected to a U. V. spectrophometer, a Waters WISP 710B automatic injector and a Perkin Elmer 56 recorder. The solvent systems used were: solvent A consisting of water and acetic acid in a ratio of 100 to 0.1, respectively; solvent B consisting of Methanol and acetic acid in a ratio of 100 to 0.1, respectively. The gradient employed was 30-50% B for 6 min, 50-75% B for 2 min, 75-100% B for 5 min, isocratic at 100% B for 5 min and then 100-30% B for 5 min. A calibration curve was prepared by injecting different volumes of a mixture of control 0.37 mg of cis- and 0.5 mg of trans-precocenediol isomers. Samples were injected into a C8 column, eluted at a flow rate of 1 mL/min and detected at 254 nm. Using this system, diols elute as a broad split peak at 16 mL while precocene elutes at 21 mL. The results are shown in Table 8.

## Table 8

| Example | Medium | Cytochrome P-450 formed (pmol/mg protein) | Precocene-diols formed (mg/mL at 48 h) |
|---|---|---|---|
| 32 | A | 122.5 | 1.27 |
| 33 | B | <2.0 | 0.23 |
| 34 | B + soybean flour | 28.0 | 0.87 |

HPLC analysis of the culture supernatants indicates the strong correlation between cytochrome P-450 concentrations and precocene-diol formation.

Example 35

Cultures of S. griseus, ATCC 13273, were grown on Medium A, on Medium B and on Medium B supplemented with genistein, and the 105,000 x g supernatant fluid collected as described in Examples 1-10. A portion of the 105,000 x g supernatant fluid from the culture grown on Medium A was then fractionated as described in Example 28. Analysis of the proteins present in the 105,000 x g supernatant fluid from the cultures grown on unsupplemented and supplemented Medium B and the 35-45% and 45-55% ammonium sulfate pellet fractions from Medium A was accomplished using sodium dodecylsulfate polyacrylamide gel electrophoresis (SDS-page). For this purpose, 20 $\mu$L of each sample of the 105,000 x g supernatant fluid, containing about 5 mg/mL of protein, was added to 10 $\mu$L of sodium dodecylsulfate solubilizing buffer and heated at 95° for 3 min. Each resulting 30 $\mu$L sample was then loaded onto a 15 x 32 cm gel containing 8% acrylamide and electrophoresed at pH 8.5, 0.1% sodium dodecyl sulfate, using a Protean slab cell (Bio-Rad) apparatus maintained at 5-10 W for 16.5 h.

In addition, cultures of a mutant strain of S. griseus which lacks the ability to synthesize P-450 enzymes were grown on Medium A, and Medium B supplemented with genistein. The supernatant was collected and the proteins present in the 105,000 x g supernatant fluid analyzed as described above.

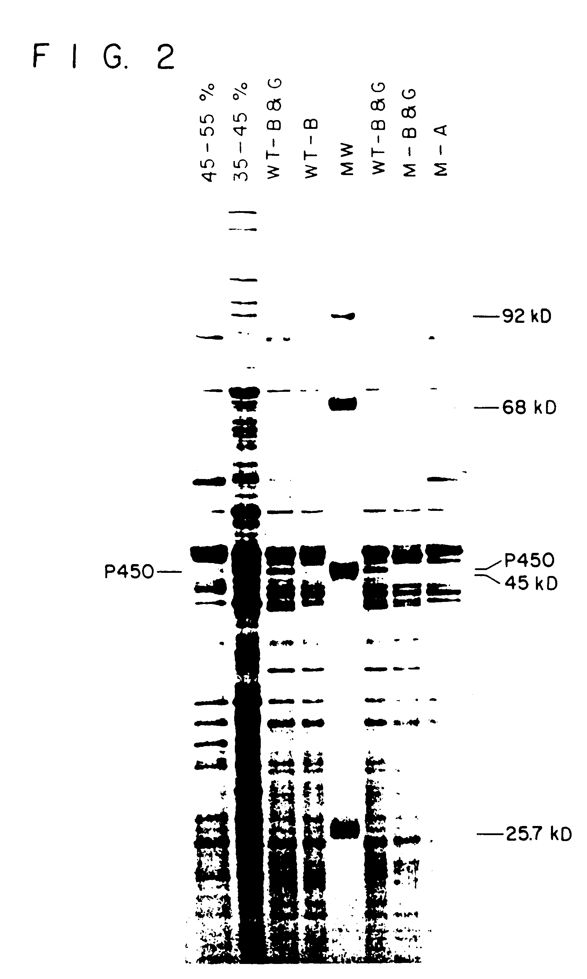

The results obtained after electrophoresis of proteins in the extracts described are shown in Fig. 2. In this figure, the lane designated MW represents the molecular weight standard. A polypeptide band with a molecular weight of 45,000 daltons was observed in extracts prepared from cells of S. griseus, ATCC 13273, grown on Medium B supplemented with genistein (lanes designated WT-B&G) but not in control cells grown on Medium B alone (lane designated WT-B). A band containing proteins of similar size was also detected in the 35-45% ammonium sulfate fractions of S. griseus, ATCC 13273, grown on Medium A (lane designated 35-45%), which, based on its relative stain intensity, appeared to have been enriched. This finding is consistent with the observation in Example 28 that P-450, as measured by CO-difference spectrophotometry, was contained primarily in the 35-45% ammonium sulfate fraction. Finally, little or none of a 45,000 molecular weight protein could be detected in the mutant strain grown on Medium A (lane designated M-A) and Medium B with genistein (lane designated M-B&G). These results lead one to

14

conclude that a 45,000 dalton peptide represents the major cytochrome P-450 protein induced by either genistein or soybean.

Example 36

Three cultures of S. griseus, ATCC 13273 were grown on Medium B as described in Examples 1-10, except that at the beginning of stage 2 growth, one of three classical mammalian P-450 inducers -- see, e.g., Bresnick et al., Pharmacological Reviews, 36:43S, (1984) (3-methylcholanthrene); Richardson et al., Cancer Res., 11:274, (1951) (3-methylcholanthrene); Conney, A.H., Pharmacol. Rev., 19:317 (1966) (phenobarbital) and Ames et al., Mutation Res., 31:347, (1975) (3-methylcholanthrene, phenobarbital and aroclor) -- were added to each of the cultures. Specifically, the inducers employed were: (1) phenobarbital added to a concentration of 0.25 mg/mL of culture; (2) 3-methylcholanthrene added to a concentration of 0.25 mg/mL of culture; and (3) aroclor added to a concentration of 1 mL/ 200 mL of culture. The bacteria were harvested after 24 h of incubation and the amount of cytochrome P-450 in each sample determined as described in Examples 1-10.

The culture containing phenobarbital produced 5.21 pmol/mg protein of cytochrome P-450 material. The culture containing 3-methylcholanthrene produced 2.72 pmol/mg protein of cytochrome P-450 material.

Example 37

Three cultures of S. griseus, ATCC 13273 were grown on Medium B as described in Examples 1-10, except that at the beginning of stage 2 growth, all three cultures received soybean flour to a concentration of 5 g/L. One culture received no further additions, one culture received metyrapone, a classic mammalian P-450 inhibitor of cytochrome P-450 induction in mammalian systems, see, e.g., Ryan et al., Methods in Enzymology, L11: 117-132 (1978), to a concentration of 0.1 mg/mL, and the remaining culture received metyrapone to a concentration of 0.25 mg/mL. The cultures were incubated an additional 24 hr before the amount of cytochrome P-450 produced by each was determined, as described in Examples 1-10. The culture with soybean flour produced 15.71 pmol of cytochrome P-450 material per mg or total protein. The cultures with metyrapone at 0.1 mg/mL and 0.25 mg/mL produced 25.1 and 38.7 pmol of cytochrome P-450 material, respectively. Clearly metyrapone did not inhibit, but rather stimulated, the production of cytochrome P-450 material, illustrating the difference between the production of mammalian and S. griseus P-450.

Example 38

Two cultures of S. griseus, ATCC 13273 were grown in Medium A essentially as described in Examples 1-10. A 200 mL stage 2 culture of each was prepared. At this time, 20 mg of Benzo[a]pyrene (BZP) which had been dissolved in 1 mL of dimethylsulfoxide was added to one of the cultures. The other culture served as a control. Both cultures were incubated, with shaking, for 24 h. The bacteria were then collected by centrifuging them at 9,000 x g for 20 min. The supernatant fluids (200 mL from each) were then extracted with 1/2 volume of ethylacetate and the resulting solutions dried under nitrogen. The dried residue from each were resuspended in 0.8 mL of dimethylsulfoxide, and 0.1 mL of the control supernatant fluids (control extract) and 0.1 mL of the BZP-treated supernatant fluids (BZP extract) was added to each of two agar plates along with the histidine requiring mutant strain of Salmonella typhimurium, strain TA 98, used in the Ames assay to detect mutagenic chemicals. In addition, 0.1 mL of the BZP-treated supernatant fluids was added to Salmonella typhimurium, strain TA 98, together with an Ames test rat microsomal S9 preparation (BZP extract + rat microsomes S9). Samples are considered to contain potential mutagenic compounds if the number of colonies--arising from a revertant mutation--on the plates containing the material being tested is two-fold or greater than the number of colonies on the plates seeded with bacteria which have not seen any additional chemicals. The latter constitute the controls and colonies arising in cultures of them represent spontaneous mutations. The results of this experiment are shown in Table 9.

## Table 9

| Extract | Revertants/plate (2 plates shown) |
|---|---|
| control extract | 166,179 |
| BZP extract | 458,425 |
| BZP extract + rat microsomes S9 | 832,755 |

These results show that S. griseus can metabolize BZP to its mutagenic forms and serve as a potential replacement for rat microsomal S9 preparations. Use of S. griseus for this test eliminates the requirements for rat microsomes with the required cofactor which are two major expenses of the Ames test as now routinely performed.

Examples 39-46

S. griseus (ATCC 13273) was grown on a soybean/glycerol medium according to the two-stage fermentation protocol. Procarcinogen substrates (20 mg/200 mL culture) were added to the 24 hr old second stage cultures and incubated at 28° for 24 hr. Incubations were then harvested by centrifuging (30 min, 10,000 x g, 4°) and both supernatants (Spt) and the pellets (P) were used for the Salmonella assay. Salmonella typhimurium strains used in these tests were TA 98 and TA 1538. The procarcinogens employed were: 2-aminoanthracene, 3,3-dimethylbenzidine, 3,3′- dimethoxybenzidine, Benzo(a)pyrene, chloropicrin, benzidine, 2-aminoacetylfluorene and 7,12-dimethylbenzanthracene. Controls in these experiments consisted of: (1) samples of cell-free supernatant fluid (Spt) and pellets (P) of S. griseus 24 hr old second stage cultures incubated without any procarcinogens (S. griseus control); (2) samples of procarcinogens in soybean/glycerol medium without S. griseus, incubated for 24 hr at 28° (procarcinogen control); and/or (3) samples of procarcinogens directly applied to the Salmonella strains (Direct Application).

Assay procedure:

The procedures for preparation of the histidine/biotin solution and top agar used, and the growth conditions employed for the Salmonella strains are discussed in the General Methods. For the assay, 500 μL of the samples to be tested (in the indicated dilutions) and 100 μL of the TA strains were mixed in tubes by vigorous shaking and incubated with shaking at 37° for 1 hr. Separately, to 75 mL of top agar was added 10 mL of histidine/biotin solution. The volume was brought up to 100 mL with sterile distilled, deionized water, and 100 μL of a 50 mg/mL solution of thiostrepton dissolved in dimethylsulfoxide (DMSO) was added. Two mL of the top agar containing the above additions was then added to the incubated tubes and the mixture poured on an agar plate. The plates were incubated at 37° for 48 hr and the number of Salmonella colonies counted.

Results of the experiments are presented in the following tables. High numbers of Salmonella revertants were observed for the procarcinogens incubated with S. griseus, indicating that S. griseus can metabolize these chemicals to mutagenic metabolites. The following abbreviations found in the Tables are defined as follows: und = undiluted; 1:2 = diluted twice; 1:10 = diluted ten times; 1:100 = diluted a hundred times; and NT = not tested.

16

## Example 39
### 2-Aminoanthracene (Procarcinogen)

|  |  | # of Salmonella revertants | |
|---|---|---|---|
|  | dilutions | TA 98 | TA 1538 |
| S. griseus control (Spt) | und | 5.5 | 2 |
|  | 1:2 | 8 | 3.5 |
|  | 1:10 | 8 | 2 |
|  | 1:100 | 7.5 | 1 |
| S. griseus control (P) | und | 7.5 | 5 |
|  | 1:2 | 14.5 | 8 |
|  | 1:10 | 12 | 11.5 |
|  | 1:100 | 6.5 | 6 |
| S. griseus test (Spt) | und | 351 | 132 |
|  | 1:2 | 195.5 | 130 |
|  | 1:10 | 49.5 | 33.5 |
|  | 1:100 | 16 | 9.5 |
| S. griseus test (P) | und | 758 | 602 |
|  | 1:2 | 1295 | 1122 |
|  | 1:10 | 582 | 555 |
|  | 1:100 | 52.5 | 66 |
| 2-aminoanthracene control (Spt) | und | 26 | 730 |
|  | 1:2 | 22.5 | 312.5 |
|  | 1:10 | 12.5 | 57.5 |
|  | 1:100 | 4.5 | 7.5 |
| 2-aminoanthracene control (P) | und | 37 | 28 |
|  | 1:2 | 61 | 21 |
|  | 1:10 | NT | 46.5 |
|  | 1:100 | NT | 54 |

Example 40

3,3'-dimethylbenzidine (Procarcinogin)

| | dilutions | # of Salmonella revertants | |
| --- | --- | --- | --- |
| | | TA 98 | TA 1538 |
| S. griseus control (Spt) | und | 22 | 3 |
| | 1:2 | 12 | 6 |
| | 1:10 | 11 | 4 |
| | 1:100 | 8 | 2 |
| S. griseus control (P) | und | 12 | 12 |
| | 1:2 | 45 | 18 |
| | 1:10 | 18 | 12 |
| | 1:100 | 13 | 6 |
| S. griseus test (Spt) | und | 1862 | 2048 |
| | 1:2 | 44 | 38 |
| | 1:10 | 17 | 10 |
| | 1:100 | 16 | 6 |
| S. griseus test (P) | und | 593 | 478 |
| | 1:2 | 216 | 331 |
| | 1:10 | 134 | 188 |
| | 1:100 | 24 | 25 |
| 3,3'-dimethylbenz- idine control (Spt) | und | 28 | 30 |
| | 1:2 | 18 | 22 |
| | 1:10 | 22 | 11 |
| | 1:100 | 19 | 8 |
| 3,3'-dimethylbenz- idine control (P) | und | 20 | 15 |
| | 1:2 | 22 | 13 |
| | 1:10 | 17 | 9 |
| | 1:100 | 13 | 8 |
| Direct Application | und | 9 | 16 |
| | 1:2 | 10 | 11 |
| | 1:10 | 16 | 8 |

## Example 41

3,3'-dimethoxybenzidine (Procarcinogen)

| | | # of Salmonella revertants | |
|---|---|---|---|
| | dilutions | TA 98 | TA 1538 |
| S. griseus control (Spt) | und | 15 | 16 |
| | 1:2 | 17 | 9 |
| | 1:10 | 17 | 2 |
| | 1:100 | 24 | 1 |
| S. griseus control (P) | und | 19 | 7 |
| | 1:2 | 26 | 15 |
| | 1:10 | 22 | 9 |
| | 1:100 | 15 | 4 |
| S. griseus test (Spt) | und | 76 | 55 |
| | 1:2 | 42 | 43 |
| | 1:10 | 32 | 14 |
| | 1:100 | 21 | 10 |
| S. griseus test (P) | und | 930 | 799 |
| | 1:2 | 259 | 300 |
| | 1:10 | 314 | 500 |
| | 1:100 | 26 | 16 |
| 3,3'-dimethoxybenz-idine control (Spt) | und | 63 | 35 |
| | 1:2 | 31 | 12 |
| | 1:10 | 25 | 6 |
| | 1:100 | 19 | 5 |
| 3,3'-dimethoxybenz-idine control (P) | und | 11 | 22 |
| | 1:2 | 12 | 14 |
| | 1:10 | 6 | 11 |
| | 1:100 | 11 | 9 |
| Direct Application | und | 12 | 15 |
| | 1:2 | 9 | 7 |
| | 1:10 | 8 | 5 |
| | 1:100 | 6 | 5 |

## Example 42

Benzo(a)pyrene (Procarcinogen)

| | dilutions | # of Salmonella revertants | |
| --- | --- | --- | --- |
| | | TA 98 | TA 1538 |
| S. griseus control (Spt) | und | 22 | 3 |
| | 1:2 | 12 | 6 |
| | 1:10 | 11 | 4 |
| | 1:100 | 8 | 2 |
| S. griseus control (P) | und | 12 | 12 |
| | 1:2 | 45 | 18 |
| | 1:10 | 18 | 12 |
| | 1:100 | 13 | 6 |
| S. griseus test (Spt) | und | 100 | 23 |
| | 1:2 | 110 | 31 |
| | 1:10 | 43 | 15 |
| | 1:100 | 9 | 10 |
| S. griseus test (P) | und | 414 | 338 |
| | 1:2 | 311 | 258 |
| | 1:10 | 233 | 206 |
| | 1:100 | 82 | 72 |
| Benzo(a)pyrene control (Spt) | und | 27 | 10 |
| | 1:2 | 29 | 8 |
| | 1:10 | 18 | 8 |
| | 1:100 | 17 | 2 |
| Benzo(a)pyrene control (P) | und | 18 | 11 |
| | 1:2 | 17 | 15 |
| | 1:10 | 12 | 8 |
| | 1:100 | 14 | 10 |

Experiment 43

Chloropicrin, 100 μl (Procarcinogen)

|  | dilutions | # of Salmonella revertants |  |
|---|---|---|---|
|  |  | TA 98 | TA 1538 |
| S. griseus control (Spt) | und | 8 | 9 |
|  | 1:2 | 8 | 5 |
|  | 1:10 | 6 | 5 |
|  | 1:100 | 5 | 4 |
| S. griseus control (P) | und | 14 | 22 |
|  | 1:2 | 26 | 19 |
|  | 1:10 | 17 | 14 |
|  | 1:100 | 17 | 6 |
| S. griseus test (Spt) | und | 115 | 33 |
|  | 1:2 | 50 | 21 |
|  | 1:10 | 13 | 10 |
|  | 1:100 | 10 | 4 |
| S. griseus test (P) | und | 72 | 29 |
|  | 1:2 | 17 | 12 |
|  | 1:10 | 10 | 7 |
|  | 1:100 | 11 | 3 |
| Chloropicrin control (Spt) | und | 19 | 14 |
|  | 1:2 | 22 | 8 |
|  | 1:10 | 9 | 3 |
|  | 1:100 | 5 | 0 |
| Chloropicrin control (P) | und | 15 | 24 |
|  | 1:2 | 10 | 5 |
|  | 1:10 | 5 | 3 |
|  | 1:100 | 12 | 4 |
| Direct Application | und | 13 | 6 |
|  | 1:2 | 11 | 4 |
|  | 1:10 | 10 | 6 |
|  | 1:100 | 11 | 6 |

## Example 44

Benzidine (Procarcinogen)

| | dilutions | # of Salmonella revertants TA 98 | TA 1538 |
|---|---|---|---|
| S. griseus control (Spt) | und | 22 | 13 |
| | 1:2 | 18 | 8.5 |
| | 1:10 | 21 | 6 |
| | 1:100 | 10 | 9.5 |
| S. griseus control (P) | und | 12 | 16.5 |
| | 1:2 | 25 | 11.5 |
| | 1:10 | 20 | 9 |
| | 1:100 | 23 | 5.5 |
| S. griseus test (Spt) | und | 246 | 315 |
| | 1:2 | 38 | 32 |
| | 1:10 | 23 | 12.5 |
| | 1:100 | 12 | 4.5 |
| S. griseus test (P) | und | 478 | 764 |
| | 1:2 | 169 | 99.5 |
| | 1:10 | 133 | 88 |
| | 1:100 | 18 | 12.5 |
| Benzidine control (Spt) | und | 53 | 17.5 |
| | 1:2 | 33 | 8 |
| | 1:10 | 18. | 7 |
| | 1:100 | 13 | 6.5 |
| Benzidine control (P) | und | 16 | 6.5 |
| | 1:2 | 13 | 4 |
| | 1:10 | 15 | 3 |
| | 1:100 | 13 | 10.5 |

## Example 45

7,12-dimethylbenzanthracene (Procarcinogen)

| | | # of Salmonella revertants | |
|---|---|---|---|
| | dilutions | TA 98 | TA 1538 |
| S. griseus control (Spt) | und | 14 | 9.5 |
| | 1:2 | 10.5 | 7.5 |
| | 1:10 | 6.5 | 4.5 |
| | 1:100 | 16 | 4.5 |
| S. griseus control (P) | und | 8.5 | 0.5 |
| | 1:2 | 26.5 | 7 |
| | 1:100 | 48.5 | 5.5 |
| S. griseus test (Spt) | und | 42.5 | 20.5 |
| | 1:2 | 41 | 13.5 |
| | 1:10 | 31 | 8 |
| | 1:100 | 34 | 8.5 |
| S. griseus test (P) | und | 39 | 15.5 |
| | 1:2 | 86.5 | 54.5 |
| | 1:10 | 103 | 108 |
| | 1:100 | 42.5 | 37 |
| 7,12-dimethylbenz- anthracene control (Spt) | und | | |

Example 46

2-Aminoacetylfluorene (Procarcinogen)

|  | dilutions | # of Salmonella revertants TA 98 | TA 1538 |
|---|---|---|---|
| S. griseus control (Spt) | und | 5.5 | 2 |
|  | 1:2 | 13 | 3.5 |
|  | 1:10 | 11 | 2 |
|  | 1:1002 | 17 | 1 |
| S. griseus control (P) | und | 11.5 | 5 |
|  | 1:2 | 17.5 | 8 |
|  | 1:10 | 20.5 | 11.8 |
|  | 1:100 | 20.5 | 6 |
| S. griseus test (Spt) | und | 93.5 | 2 |
|  | 1:2 | 56 | 4 |
|  | 1:10 | 30 | 7 |
|  | 1:100 | 17.5 | 7 |
| S. griseus test (P) | und | 5000 | 299 |
|  | 1:2 | 1175 | 262.5 |
|  | 1:10 | 467.5 | 67.5 |
|  | 1:100 | 322.5 | 11 |
| 2-aminoacetyl-fluorene control (Spt) | und | NT | 4 |
|  | 1:2 | NT | 8.5 |
|  | 1:10 | NT | 4 |
|  | 1:100 | NT | 0.5 |
| 2-aminoacetyl-fluorene control (P) | und | NT | 26 |
|  | 1:2 | NT | 21 |
|  | 1:10 | NT | 12 |
|  | 1:100 | NT | 6 |

**Claims**

1. A method for evaluating the potential mutagenic activity of a substance which comprises:
   (a) inducing the production of cytochrome P-450 in Streptomyces griseus by culturing said bacteria in a medium containing soybean flour or a soybean derivative;
   (b) culturing a sample of Streptomyces griseus bacteria obtained from step (a) in a culture medium comprising the substance to be evaluated;
   (c) incubating an amino acid requiring mutant strain of Salmonella typhimurium in the presence of a sample of bacterial culture from (b); and
   (d) determining the number of resulting amino acid sufficient revertant colonies.

2. The method of claim 1 further comprising centrifuging the bacterial culture after step (b) and prior to step (c) to obtain a cellular pellet portion and a substantially cell-free supernatant portion.

3. The method of claim 2 wherein the bacterial culture sample incubated with the Salmonella strain in step (c) is taken from the cellular pellet portion.

4. The method of claim 2 wherein the bacterial culture sample incubated with the Salmonella strain in step (c) is taken from the cell-free supernatant portion.

EP 0 273 771 B1

5. The method of any of claims 2 to 4 wherein the centrifuging is carried out at a rate of 8,000 to 15,000 x g for 15 to 30 minutes.

6. The method of claim 5 wherein the centrifuging is carried out at a rate of about 9,000 x g for about 20 minutes.

7. The method of any of claims 1 to 6 wherein the bacteria in step (a) are selected from Streptomyces griseus ATCC No. 13273, Streptomyces griseus ATCC No. 10137, Streptomyces griseus ATCC No. 13968, Streptomyces griseus ATCC No. 21897, Streptomyces griseus NRRL No. B-8090, Streptomyces punipalus NRRL No. 3529 and Streptomyces griseolus ATCC No. 11796.

8. The method of claim 7 wherein the bacteria in step (a) are selected from Streptomyces griseus ATCC No. 13273, Streptomyces griseus ATCC No. 10137, Streptomyces griseus ATCC No. 13968, Streptomyces griseus NRRL No. B-8090, Streptomyces punipalus NRRL No. 3529 and Streptomyces griseolus ATCC No. 11796.

9. The method of claim 8 wherein the bacteria in step (a) are selected from Streptomyces griseus ATCC No. 13273, Streptomyces griseus ATCC No. 10137 and Streptomyces griseus NRRL No. B-8090.

10. The method of claim 9 wherein the bacteria in step (a) is Streptomyces griseus ATCC No. 13273.

11. The method of any of the preceding claims wherein the amino acid requiring mutant strain of Salmonella typhimurium is a histidine requiring mutant strain.

12. The method of claim 11 wherein the histidine requiring mutant strain of Salmonella typhimurium is selected from strains TA 97, TA 97A, TA 98, TA 100, TA 100FR, TA 102, TA 104, TA 1535, TA 1537, TA 1538, and M 677.

13. The method of claim 12 wherein the mutant strain of Salmonella typhimurium is selected from strains TA 98, TA 1535, TA 1537 and TA 1538.

14. The method of claim 13 wherein the mutant strain of Salmonella typhimurium is selected from strains TA 1535, TA 1537 and TA 1538.

15. The method of any of the preceding claims wherein said soybean derivative comprises genistein or genistin.

**Patentansprüche**

1. Verfahren zur Bestimmung der potentiellen mutagenen Aktivität einer Substanz, umfassend:
   a) Das Induzieren der Bildung von Cytochrom P-450 in Streptomyces griseus durch Kultivieren der Bakterien in einem Medium, das Sojabohnenmehl oder ein Sojabohnenderivat enthält;
   b) das Kultivieren einer Probe von nach Schritt (a) erhaltenen Streptomyces griseus Bakterien in einem Kulturmedium, das die zu bestimmende Substanz umfaßt;
   (c) das Inkubieren eines eine Aminosäure erfordernden Mutantenstamms von Salmonella typhimurium in Gegenwart einer Probe der Bakterienkultur aus (b); und
   (d) das Bestimmen der Anzahl der resultierenden, Aminosäure nicht erfordernden, Revertantenkolonien.

2. Verfahren nach Anspruch 1, weiter umfassend das Zentrifugieren der Bakterienkultur nach dem Schritt (b) und vor dem Schritt (c), um einen Zellpelletanteil und einen im wesentlichen zellfreien, überstehenden Anteil zu erhalten.

3. Verfahren nach Anspruch 2, worin die Bakterienkulturprobe, die mit dem Salmonellastamm im Schritt (c) inkubiert ist, dem Zellpelletanteil entnommen wird.

4. Verfahren nach Anspruch 2, worin die Bakterienkulturprobe, die mit dem Salmonellastamm im Schritt (c) inkubiert ist, dem zellfreien, überstehenden Anteil entnommen wird.

25

**5.** Verfahren nach irgendeinem der Ansprüche 2 bis 4, worin das Zentrifugieren 15 bis 30 Minuten mit einer Geschwindigkeit von 8 000 bis 15 000 x g durchgeführt wird.

**6.** Verfahren nach Anspruch 5, worin das Zentrifugieren etwa 20 Minuten bei einer Geschwindigkeit von etwa 9 000 x g durchgeführt wird.

**7.** Verfahren nach irgendeinem der Ansprüche 1 bis 6, worin die Bakterien in Schritt (a) aus Streptomyces griseus ATCC Nr. 13273, Streptomyces griseus ATCC Nr. 10137, Streptomyces griseus ATCC Nr. 13968, Streptomyces griseus ATCC Nr. 21897, Streptomyces griseus NRRL Nr. B-8090, Streptomyces punipalus NRRL Nr. 3529 und Streptomyces griseolus ATCC Nr. 11796 ausgewählt sind.

**8.** Verfahren nach Anspruch 7, worin die Bakterien in Schritt (a) aus Streptomyces griseus ATCC Nr. 13273, Streptomyces griseus ATCC Nr. 10137, Streptomyces griseus ATCC Nr. 13968, Streptomyces griseus NRRL Nr. B-8090, Streptomyces punipalus NRRL Nr. 3529 und Streptomyces griseolus ATCC Nr. 11796 ausgewählt sind.

**9.** Verfahren nach Anspruch 8, worin die Bakterien in Schritt (a) aus Streptomyces griseus ATCC Nr. 13273, Streptomyces griseus ATCC Nr. 10137 und Streptomyces griseus NRRL Nr. B-8090 ausgewählt sind.

**10.** Verfahren nach Anspruch 9, worin die Bakterien in Schritt (a) Streptomyces griseus ATCC Nr. 13273 sind.

**11.** Verfahren nach irgendeinem der vorhergehenden Ansprüche, worin der eine Aminosäure erfordernde Mutantenstamm von Salmonella typhimurium ein Histidin erfordernder Mutantenstamm ist.

**12.** Verfahren nach Anspruch 11, worin der Histidin erfordernde Mutantenstamm von Salmonella typhimurium aus den Stämmen TA 97, TA 97A, TA 98, TA 100, TA 100FR, TA 102, TA 104, TA 1535, TA 1537, TA 1538 und M 677 ausgewählt ist.

**13.** Verfahren nach Anspruch 12, worin der Mutantenstamm von Salmonella typhimurium aus den Stämmen TA 98, TA 1535, TA 1537 und TA 1538 ausgewählt ist.

**14.** Verfahren nach Anspruch 13, worin der Mutantenstamm von Salmonella typhimurium aus den Stämmen TA 1535, TA 1537 und TA 1538 ausgewählt ist.

**15.** Verfahren nach irgendeinem der vorhergehenden Ansprüche, worin das Sojabohnenderivat Genistein oder Genistin umfaßt.

**Revendications**

**1.** Un procédé d'évaluation de l'activité mutagène potentielle d'une substance qui comprend:
(a) l'induction de la production du cytochrome P-450 chez Streptomyces griseus par culture de ladite bactérie dans un milieu contenant une farine de soja ou un dérivé de soja;
(b) la culture d'un échantillon d'une bactérie Streptomyces griseus obtenue à partir de l'étape (a) dans un milieu de culture comprenant la substance devant être évaluée;
(c) l'incubation d'une souche mutante de Salmonella typhimurium nécessitant un acide aminé en présence d'un échantillon de la culture bactérienne provenant de (b); et
(d) la détermination du nombre de colonies révertantes pour l'acide aminé qui en résulte.

**2.** Le procédé selon la revendication 1, comprenant de plus la centrifugation de la culture bactérienne après l'étape (b) et avant l'étape (c) pour obtenir une partie de concentrat cellulaire et une partie de surnageant substantiellement exempt de cellule.

**3.** Le procédé selon la revendication 2, caractérisé en ce que l'échantillon de culture bactérienne incubé avec la souche de Salmonella dans l'étape (c) provient de la partie correspondant au concentrat cellulaire.

**4.** Le procédé selon la revendication 2, caractérisé en ce que l'échantillon de culture bactérienne incubé avec la souche de Salmonella dans l'étape (c) provient de la partie surnageante exempte de cellule.

**5.** Le procédé selon l'une quelconque des revendications 2 à 4, caractérisé en ce que la centrifugation est mise en oeuvre à une vitesse comprise entre 8 000 et 15 000 x g pendant 15 à 30 minutes.

**6.** Le procédé selon la revendication 5, caractérisé en ce que la centrifugation est mise en oeuvre à une vitesse d'environ 9 000 x g pendant environ 20 minutes.

**7.** Le procédé selon l'une quelconque des revendications 1 à 6, caractérisé en ce que la bactérie dans l'étape (a) est sélectionnée dans le groupe consistant en Streptomyces griseus ATCC n° 13273, Streptomyces griseus ATCC n° 10137, Streptomyces griseus ATCC n° 13968, Streptomyces griseus ATCC n° 21897, Streptomyces griseus NRRL n° B-8090, Streptomyces punipalus NRRL n° 3529 et Streptomyces griseolus ATCC n° 11796.

**8.** Le procédé selon la revendication 7, caractérisé en ce que la bactérie de l'étape (a) est sélectionnée dans le groupe consistant en Streptomyces griseus ATCC n° 13273, Streptomyces griseus ATCC n° 10137, Streptomyces griseus ATCC n° 13968, Streptomyces griseus NRRL n° B-8090, Streptomyces punipalus NRRL n° 3529 et Streptomyces griseolus ATCC n° 11796.

**9.** Le procédé selon la revendication 8, caractérisé en ce que la bactérie dans l'étape (a) est sélectionnée dans le groupe consistant en Streptomyces griseus ATCC n° 13273, Streptomyces griseus ATCC n° 10137 et Streptomyces griseus NRRL n° B-8090.

**10.** Le procédé selon la revendication 9, caractérisé en ce que la bactérie dans l'étape (a) est Streptomyces griseus ATCC n° 13273.

**11.** Le procédé selon l'une quelconque des revendications précédentes, caractérisé en ce que la souche mutante de Salmonella typhimurium nécessitant un acide aminé est une souche mutante qui nécessite de l'histidine.

**12.** Le procédé selon la revendication 11, caractérisé en ce que la souche mutante de Salmonella typhimurium nécessitant de l'histidine est sélectionnée parmi les souches TA 97, TA 97A, TA 98, TA 100, TA 100FR, TA 102, TA 104, TA 1535, TA 1537, TA 1538 et M 677.

**13.** Le procédé selon la revendication 12, caractérisé en ce que la souche mutante de Salmonella typhimurium est sélectionnée parmi les souches TA 98, TA 1535, TA 1537 et TA 1538.

**14.** Le procédé selon la revendication 13, caractérisé en ce que la souche mutante de Salmonella typhimurium est sélectionnée parmi les souches TA 1535, TA 1537 et TA 1538.

**15.** Le procédé selon l'une quelconque des revendications précédentes, dans lequel ledit dérivé de soja comprend de la génistéine ou de la génistine.

# F I G. 1A

P 450

Time [min]

# F I G. 1B

P 450

Time [min]

F I G. 2